# EUROPÄISCHE PATENTSCHRIFT

(11) **EP 1 028 733 B1**
(45) Veröffentlichungstag und Bekanntmachung des Hinweises auf die Patenterteilung: **06.04.2005**
(21) Anmeldenummer: 98959848.7
(22) Anmeldetag: 05.11.1998
(51) Int. Cl.: A61K 31/575, A61P 3/00

(54) **VERWENDUNG VON PHYTOSTENOL ENTHALTENDER WIRKSTOFFMISCHUNGEN ZUR HERSTELLUNG VON HYPOCHOLESTERINÄMISCHEN MITTELN**
USE OF MIXTURES OF ACTIVE AGENTS CONTAINING PHYTOSTENOL FOR PRODUCING HYPOCHOLESTERAEMIC PREPARATIONS
UTILISATION DE MELANGES DE PRINCIPES ACTIFS CONTENANT DU PHYTOSTENOL POUR LA PRODUCTION D'AGENTS HYPOCHOLESTERINEMIQUES

(30) Priorität: 14.11.1997 DE 19750453
(43) Veröffentlichungstag der Anmeldung: 23.08.2000
(73) Patentinhaber: Cognis Deutschland GmbH & Co. KG, 40589 Düsseldorf (DE)
(72) Erfinder: FABRY, Bernd, D-41352 Korschenbroich (DE)
(86) Internationale Anmeldenummer: PCT/EP1998/007059
(87) Internationale Veröffentlichungsnummer: WO 1999/025362

(56) Entgegenhaltungen:
- WO-A-98/03084
- WO-A-98/33494
- DE-A- 2 408 067
- CHEMICAL ABSTRACTS, vol. 100, no. 25, 18. Juni 1984 Columbus, Ohio, US; abstract no. 208354, HASEGAWA ET AL: "Hypocholesteraemic Effect of Linoleic Acid and Phytosterol" XP002099834 & HASEGAWA ET AL: JOSHI EIYO DAIGAKU KIYO, Bd. 14, 1983, Seiten 165-172,
- CHEMICAL ABSTRACTS, vol. 93, no. 5, 4. August 1980 Columbus, Ohio, US; abstract no. 41530, FUNES ET AL: XP002099835 & FUNES, CATTANEO: "Studies on the Prunus (Rosaceae) Seeds of Argentine Production. I. Oils of the Seed of Peach, Apricot, Plum, Cherry etc." AN. ASOC. QUIM. ARGENT., Bd. 66, Nr. 5, 1978, Seiten 239-253,

## Beschreibung

### Gebiet der Erfindung

Die Erfindung betrifft die Verwendung von synergistischen Mischungen aus β-Sitostenolestern und konjugierten Fettsäuren zur Herstellung von Mitteln zur Verminderung des Cholesteringehaltes im Serum von Warmblütern.

### Stand der Technik

Unter hypochloesterinämischen Wirkstoffen werden Mittel verstanden, die zu einer Verminderung des Cholesteringehaltes im Serum von Warmblütern führen, ohne daß dadurch eine Hemmung oder Verringerung der Bildung von Cholesterin im Blut eintritt. Für diesen Zweck wurden bereits von Peterson et al. in **J.Nutrit.** **50****, 191 (1953)** Phytostenole, also pflanzliche Stenole, und deren Ester mit Fettsäuren vorgeschlagen. In die gleiche Richtung weisen auch die Patentschriften **US 3,089,939, US 3,203,862** sowie die deutsche Offenlegungsschrift **DE-OS 2035069** (Procter & Gamble). Die Wirkstoffe werden üblicherweise Brat- oder Speiseölen zugesetzt und dann über die Nahrung aufgenommen, wobei die Einsatzmengen jedoch in der Regel gering sind und üblicherweise unter 0,5 Gew.-% liegen, um zu verhindern, daß die Speiseöle eintrüben oder die Stenole bei Zusatz von Wasser ausgefällt werden. Für den Einsatz im Nahrungsmittelbereich, in Kosmetika, pharmazeutischen Zubereitungen und im Agrarsektor werden in der europäischen Patentanmeldung **EP-A1 0289636** (Ashai) lagerstabile Emulsionen der Stenolester in Zucker- oder Polyglycerinestern vorgeschlagen. Die Einarbeitung von Sitostanolestern zur Verminderung des Blutcholesteringehaltes in Margarine, Butter, Mayonnaise, Salatsaucen und dergleichen wird in der Europäischen Patentschrift **EP-B1 0594612** (Raision) vorgeschlagen. In der Internationalen Patentanmeldung **WO-A-98 33494** (J.V. Kosbab) werden Formulierungen vorgeschlagen, die Phytosterole in Kombination mit Linolsäure zur Inhibierung der Cholesterinabsorption aufführen. Ebenso wird von Hasegawa et al. in **Joshi Eiyo Daigaku Kiyo Vol. 14, Seiten 165 -172** beschrieben, dass Phytosterol und Linolsäure eine cholesterinsenkende Wirkung aufweisen.

Von Nachteil ist jedoch, daß die Phytostenolester den Nahrungsmitteln üblicherweise nur in geringen Mengen zugesetzt werden können, da ansonsten die Gefahr besteht, daß sie den Geschmack und/oder die Konsistenz der Mittel beeinträchtigen. Zur nachhaltigen Beeinflussung des Cholesteringehaltes im Blut wäre jedoch die Aufnahme größerer Mengen Phytostenole bzw. Phytostenolester wünschenswert. Weiterhin verbesserungswürdig ist die Geschwindigkeit, mit der die Stoffe den Gehalt an Cholesterin im Serum vermindern. Die Aufgabe der Erfindung hat folglich darin bestanden, diesen Mängeln abzuhelfen.

### Beschreibung der Erfindung

Gegenstand der Erfindung ist die Verwendung von Wirkstoffmischungen zur Herstellung von hypocholesterinämischen Mitteln mit der Maßgabe, dass man
(a) Ester von β-Sitostenol bzw. β-Sitostanol mit Carbonsäuren der Formel (I),

   **R**^{**1**}**CO-OH** **(I)**

   in der R¹CO für einen aliphatischen, linearen oder verzweigten Acylrest mit 2 bis 22 Kohlenstoffatomen und 0 und/oder 1, 2 oder 3 Doppelbindungen steht,
   und
(b) Fettsäuren mit 6 bis 24 Kohlenstoffatomen und mindestens zwei konjugierten Doppelbindungen bzw. deren Glyceride
einsetzt.

Überraschenderweise wurde gefunden, daß Mischungen von den beschriebenen β-Sitostenolestern (Komponente a) mit konjugierten Fettsäuren bzw. Fettsäureglyceriden (Komponente b) in synergistischer Weise die Verminderung des Cholesteringehaltes im Blutserum bewirken. In Gelatine verkapselt oder Nahrungsmitteln direkt zugesetzt, lassen sich sowohl die Wirkstoffgemische problemlos oral einnehmen.

### Phytostenole und Phytostenolester

Unter Phytostenolen (oder synonym Phytosterolen) sind pflanzliche Steroide zu verstehen, die nur am C-3 eine Hydroxylgruppe, sonst aber keine funktionellen Gruppen tragen. In der Regel besitzen die Phytostenole 27 bis 30 Kohlenstoffatome und eine Doppelbindung in 5/6, gegebenenfalls 7/8, 8/9 oder anderen Positionen. Neben diesen ungesättigten Spezies kommen als Stenole auch die durch Härtung erhältlichen gesättigten Verbindungen in Frage, die als Stanole bezeichnet werden und von der vorliegenden Erfindung mitumschlosen werden. Typische Beispiele für geeignete Phytostenole sind beispielsweise Ergostenole, Campestenole, Stigmastenole, Brassica-stenole sowie vorzugsweise Sito-stenole bzw. Sitostanole und insbesondere β-Sitostenole bzw. β-Sitostanole. Der genannten Phytostenolen werden als. Ester eingesetzt. Die Säurekomponente der Esters geht auf Carbonsäuren der Formel (I) zurück,

**R**^{**1**}**CO-OH** **(I)**

in der R¹CO für einen aliphatischen, linearen oder verzweigten Acylrest mit 2 bis 22 Kohlenstoffatomen und 0 und/oder 1, 2 oder 3 Doppelbindungen steht. Typische Beispiele sind Essigsäure, Propionsäure, Buttersäure, Valeriansäure, Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkemoder Talgfettsäure. Besonders bevorzugt ist der Einsatz von Estern des β-Sitostenols bzw. β-Sitostanols mit Fettsäuren mit 12 bis 18 Kohlenstoffatomen. Diese Ester können sowohl durch direkte Veresterung der Phytostenole mit den Fettsäuren oder aber durch Umesterung mit Fettsäureniedrigalkylestem oder Triglyceriden in Gegenwart geeigneter Katalysatoren, wie z.B. Natriumethylat oder speziell auch Enzymen hergestellt werden [vgl. **EP.A2 0195311** (Yoshikawa)]. Die hypocholesterinämische Wirkung von Phytostenolen bzw. Phytostenolestem ist beispielsweise aus der Europäischen Patentschrift **EP-B1 0594612** (Raision) sowie der dort zitierten Literatur bekannt.

### Konjugierte Fettsäuren

Unter dem Begriff konjugierte Fettsäuren versteht man aliphatische Carbonsäuren mit 6 bis 24, vorzugsweise 16 bis 18 Kohlenstoffatomen und mindestens zwei Doppelbindungen, die sich zueinander in Konjugation befinden, d.h. durch genau eine Einfachbindung voneinander getrennt sind. Typische Beispiele sind die konjugierte Linolsäure (CLA) oder konjugierte Fischfettsäuren. Von konjugierter Linolsäure ist bekannt, daß sie eine geringe hypocholesterinämische Wirkung besitzt; ihre Verwendung in Lebensmitteln oder als Lebensmittelergänzungsstoff geht aber darauf zurück, daß sie die Verbrennung von körpereigenen Fetten unterstützt [vgl. **EP-B1 0579901, WO 94/16690, WO 96/06605**; (WARF)]. Anstelle der konjugierten Fettsäuren können aus geschmacklichen Gründen und wegen der besseren Fettlöslichkeit auch die entsprechenden Voll- oder Partialester mit Glycerin eingesetzt werden.

### Tocopherole

Als weitere Bestandteile können die Wirkstoffgemische Potenzierungsmittel vom Typ der Tocopherole enthalten. Unter Tocopherolen werden in 2-Stellung mit 4,8,12-Trimethyltridecyl-Resten substituierte Chroman-6-ole (3,4-Dihydro-2-*H*-1benzopyran-6-ole) verstanden, die der Formel **(II)** folgen, 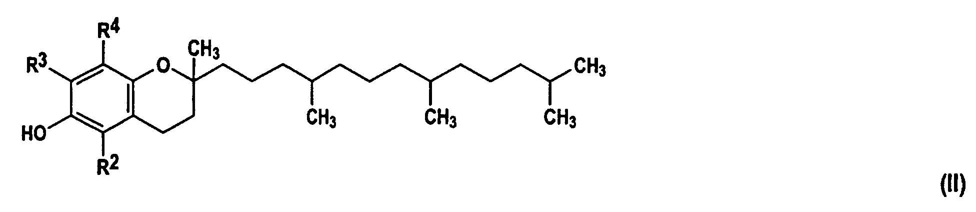 in der R², R³ und R⁴ unabhängig voneinander für Wasserstoff oder eine Methylgruppe stehen. Tocopherole zählen zu den Biochinonen, also polyprenylierten 1,4-Benzo- bzw. Naphthochinonen, deren Prenylketten mehr oder weniger stark gesättigt sind. Typische Beispiele für Tocopherole, die im Sinne der Erfindung als Komponente (b) in Betracht kommen, sind Ubichinone, Bovichinone, K-Vitamine und/oder Menachinone (2-Methyl-1,4-Naphthochinone). Man unterscheidet bei den Tocopherolen weiterhin α, β, γ-, δ- und ε-Tocopherole, wobei letztere noch über die ursprüngliche ungesättigte Prenylseitenkette verfügen, sowie α-Tocopherolchinon und -hydrochinon, bei denen das Pyran-Ringsystem geöffnet ist. Vorzugsweise wird als Komponente (b) α-Tocopherol (Vitamin E) der Formel (II) eingesetzt, bei der R², R³ und R⁴ für Methylgruppen stehen, oder Ester des α-Tocopherols mit Car bonsäuren mit 2 bis 22 Kohlenstoffatomen, wie beispielsweise α-Tocopherolacetat oder α-Tocopherolpalmitat.

### Chitosane

Als weitere Bestandteile können die Wirkstoffgemische Potenzierungsmittel vom Typ der Chitosane enthalten. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein **(III)** enthalten:

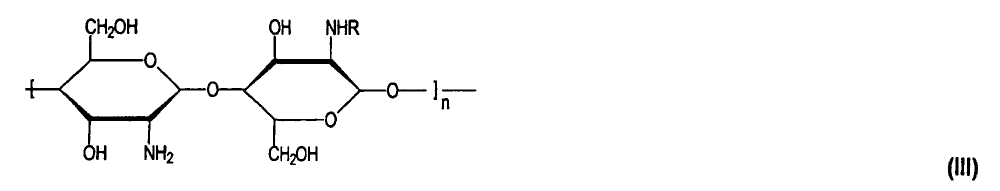

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim**, **Verlag Chemie, 1986, S. 231-332).** Übersichten zu diesem Thema sind auch beispielsweise von B.Gesslein et al. in **HAPPI** **27,** **57 (1990)**, O.Skaugrud in **Drug Cosm.Ind.** **148,** **24 (1991)** und E.Onsoyen et al. in **Seifen-Öle-Fette-Wachse** **117,** **633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol.Chem.** **177,** **3589 (1976)** oder der französischen Patentanmeldung **FR-A 2701266** bekannt. In einer bevorzugten Ausführungsform der Erfindung wird ein Chitinabbauprodukt, wie es in der Internationalen Patentanmeldung WO 96/16991 (Henkel) beschrieben wird, oder dessen Abbauprodukt mit Wasserstoffperoxid eingesetzt.

### Phytostenolsulfate

Als weitere Bestandteile können die Wirkstoffgemische Potenzierungsmittel vom Typ der Phytostenolsulfate enthalten. Phytostenolsulfate stellen bekannte Stoffe dar, die beispielsweise durch Sulfatierung von Phytostenolen mit einem Komplex aus Schwefeltrioxid und Pyridin in Benzol hergestellt werden können [vgl. **J.Am. Chem.Soc.** **63,** **1259 (1941)**]. Typische Beispiele sind die Sulfate von Ergostenolen, Campestenolen, Stigmastenolen und Sitostenolen. Die Phytostenolsulfate können als Alkali- und/oder Erdalkalimetallsalze, als Ammonium-, Alkylammonium-, Alkanolammonium- und/oder Glucammoniumsalze vorliegen. In der Regel werden sie in Form ihrer Natriumsalze eingesetzt.

### (Desoxy)Ribonucleinsäuren

Als weitere Bestandteile können die Wirkstoffgemische schließlich Potenzierungsmittel vom Typ der (Desoxy)Ribonucleinsäuren enthalten. Unter (Desoxy)Ribonucleinsäuren (DNA bzw. RNA) werden hochmolekulare, fadenförmige Polynucleotide verstanden, die sich von 2'-Desoxy-β-D-ribonucleosiden bzw. D-Ribonucleosiden ableiten, die ihrerseits wieder von äquivalenten Mengen einer Nucleobase und der Pentose 2-Desoxy-D-ribo-furanose bzw. D-Ribofuranose aufgebaut werden. Als Nucleobasen können die DNA bzw. RNA die Purinderivate Adenin und Guanin sowie die Pyrimidine Cytosin und Thymin bzw. Uracil enthalten. In den Nucleinsäuren sind die Nucleobasen N-glykosidisch mit Kohlenstoffatom 1 der Ribose, wodurch im Einzelfall Adenosine, Guanosine, Cytidine und Thimidine entstehen. In den Säuren verknüpft eine Phosphatgruppe die 5'-Hydroxygruppe der Nucleoside mit der 3'-OH-Gruppe der jeweils folgenden durch eine Phosphodiesterbrücke unter Ausbildung von Einzelstrang-DNA bzw. -RNA. Wegen des großen Verhältnisses von Länge zu Durchmesser neigen DNA- bzw. RNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton reichen. Im Sinne der Erfindung werden konzentrierte DNA bzw. RNA-Lösungen eingesetzt, die sich durch ein flüssigkristallines Verhalten auszeichnen. Vorzugsweise werden Desoxy- bzw. Ribonucleinsäuren eingesetzt, die aus marinen Quellen beispielsweise durch Extraktion von Fischsperma erhalten werden und die ein Molekulargewicht im Bereich von 40.000 bis 1.000.000 Dalton aufweisen.

### Gewerbliche Anwendbarkeit

Die Wirkstoffmischungen der Erfindung können die Phytostenole und/oder Phytostenolester und die konjugierten Fettsäuren im Gewichtsverhältnis 99 : 1 bis 1 : 99, vorzugsweise 90 : 10 bis 10 : 90, insbesondere 70 : 25 bis 25 : 75 und besonders bevorzugt 60 : 40 bis 40 : 60 enthalten. In einer besonderen Ausführungsform der Erfindung werden die Wirkstoffmischungen in an sich bekannter Weise in Gelatine verkapselt, wobei man die Komponenten (a) und (b) jeweils in Mengen von 0,1 bis 50, vorzugsweise 1 bis 30, insbesondere 5 bis 25 und besonders bevorzugt 10 bis 15 Gew.-% - bezogen auf das Gewicht der Gelatinekapseln - einsetzt. Daneben ist es möglich, die Mischungen in üblichen Nahrungsmitteln zu lösen bzw. zu dispergieren, als da beispielsweise sind : Butter, Margarine, Diätnahrung, Fritieröle, Speiseöle, Mayonnaisen, Salatdressings, Kakaoprodukte, Wurst und dergleichen.

### Beispiele

### Beispiele 1 bis 5, Vergleichsbeispiele V1 bis V7

Es wurden Gelatinekapseln (Gewicht ca. 1,5 g) mit einem Gehalt von 5 bzw. 10 Gew.-% β-Sitostenol bzw. β-Sitostenolester und gegebenenfalls 5 bzw. 10 Gew.-% konjugierte Linolsäure (CLA) sowie 0,5 Gew.-% radioaktiv markiertem Cholesterin hergestellt. Zur Untersuchung der hypocholesterinämischen Wirkung ließ man männliche Ratten (Einzelgewicht ca. 200 g) über Nacht fasten. Am folgenden Tag wurde den Versuchstieren jeweils eine zerkleinerte Gelatinekapsel mit etwas kochsalzhaltigem Wasser über eine Magensonde eingeführt. Nach 3, 6, 12, 24 und 48 h wurde den Tieren Blut abgenommen und der Gehalt an radioaktivem Cholesterin bestimmt. Die Ergebnisse, die den Mittelwert der Messungen von 10 Versuchstieren darstellen, sind in Tabelle 1 zusammengefaßt. Die Angaben zur Abnahme der Radioaktivität verstehen sich jeweils in Bezug auf eine Blindgruppe von Versuchstieren, denen lediglich Gelatinekapseln mit einem Gehalt von 20 Gew.-% Vitamin E und einer entsprechenden Menge radioaktiv markiertem Cholesterin verabreicht worden war. Die Mischungen 3 bis 5 sind erfindungsgemäß, die Mischungen V1 bis V7 dienen dem Vergleich.

**Tabelle 1**

| **Hypocholesterinämische Wirkung (Mengenangaben als Gew.-% bezogen auf Gelatinekapsel)** | | | | | | | | | | |
|---|---|---|---|---|---|---|---|---|---|---|
| **Zusammensetzung** | **V6** | **V7** | **3** | **4** | **5** | **V1** | **V2** | **V3** | **V4** | **V5** |
| β-Sitostenol | 5 | - | - | - | - | 10 | - | - | - | - |
| β-Sitostanol | - | 5 | - | - | - | - | 10 | - | - | - |
| Laurinsäure-β-sitostenolester | - | - | 5 | - | - | - | - | 10 | - | - |
| Laurinsäure-β-sitostanolester | - | - | - | 5 | 10 | - | - | - | 10 | - |
| Konjugierte Linolsäure | 5 | 5 | 5 | 5 | 5 | - | - | - | - | 10 |
| ***Radioaktivität [%-rel**]* | | | | | | | | | | |
| ***- nach 3 h*** | 93 | 93 | 93 | 93 | 93 | 93 | 93 | 93 | 93 | 98 |
| **-** ***nach 6 h*** | 84 | 83 | 83 | 83 | 81 | 87 | 86 | 87 | 86 | 91 |
| ***- nach 12h*** | 75 | 75 | 75 | 74 | 71 | 79 | 79 | 78 | 78 | 87 |
| **-** ***nach 24 h*** | 54 | 51 | 47 | 45 | 40 | 62 | 60 | 59 | 69 | 75 |
| ***- nach 48 h*** | 23 | 21 | 22 | 19 | 12 | 35 | 32 | 35 | 32 | 60 |

Die Beispiele zeigen die synergistische Abnahme des Cholesteringehaltes im Blut bei Einsatz von Mischungen der Stenole bzw. Stenolester mit CLA.

## Patentansprüche

1. Verwendung von Wirkstoffmischungen zur Herstellung von hypocholesterinämischen Mitteln, **dadurch gekennzeichnet, dass** man
(a) Ester von β-Sitostenol bzw. β-Sitostanol mit Carbonsäuren der Formel **(I)**,
**R**^{**1**}**CO-OH** **(I)**
in der R¹CO für einen aliphatischen, linearen oder verzweigten Acylrest mit 2 bis 22 Kohlenstoffatomen und 0 und/oder 1, 2 oder 3 Doppelbindungen steht,
und
(b) Fettsäuren mit 6 bis 24 Kohlenstoffatomen und mindestens zwei konjugierten Doppelbindungen bzw. deren Glyceride
einsetzt.

2. Verwendung nach Anspruch 1, **dadurch gekennzeichnet, dass** man als Komponente (a) Ester von β-Sitostenol bzw. β-Sitostanol mit Fettsäuren mit 12 bis 18 Kohlenstoffatomen ein-setzt.

3. Verwendung nach den Ansprüchen 1 und/oder 2, **dadurch gekennzeichnet, dass** man als Komponente (b) konjugierte Linolsäure (CLA) einsetzt.

4. Verwendung nach den Ansprüchen 1 bis 3, **dadurch gekennzeichnet, dass** man die Komponenten (a) und (b) im Gewichtsverhältnis 99: 1 bis 1 : 99 einsetzt.

5. Verwendung nach den Ansprüchen 1 bis 4, **dadurch gekennzeichnet, dass** man die Komponenten (a) und (b) in Gelatine verkapselt.

6. Verwendung nach Anspruch 5, **dadurch gekennzeichnet, dass** man die Komponenten (a) und (b) jeweils in Mengen von 0,1 bis 50 Gew.-% - bezogen auf das Gewicht der Gelatinekapseln - einsetzt.

7. Verwendung nach den Ansprüchen 1 bis 4, **dadurch gekennzeichnet, dass** man die Komponenten (a) und (b) Lebensmitteln zusetzt.

8. Verwendung nach Anspruch 1, **dadurch gekennzeichnet, dass** man die Komponenten (a) und (b) in Butter, Margarine, Diätnahrung, Fritierölen, Speiseölen, Mayonnaisen, Salatdressings, Kakaoprodukten, Wurst und dergleichen dispergiert.

## Claims

1. Use of mixtures of active agents for producing hypocholesteremic preparations, **characterized in that** it comprises employing
(a) esters of β-sitostenol or β-sitostanol with carboxylic acids of the formula (I)
**R**^{**1**}**CO-OH** **(I)**
in which R¹CO is an aliphatic, linear or branched acyl radical having 2 to 22 carbon atoms and 0 and/or 1, 2 or 3 double bonds
and
(b) fatty acids having 6 to 24 carbon atoms and at least two conjugated double bonds or their glycerides.

2. Use according to Claim 1, **characterized in that**, as component (a), esters of β-sitostenol or β-sitostanol with fatty acids having 12 to 18 carbon atoms are employed.

3. Use according to Claims 1 and/or 2, **characterized in that**, as component (b), conjugated linoleic acid (CLA) is employed.

4. Use according to Claims 1 to 3, **characterized in that** components (a) and (b) are employed in the weight ratio 99:1 to 1:99.

5. Use according to Claims 1 to 4, **characterized in that** components (a) and (b) are encapsulated in gelatin.

6. Use according to Claim 5, **characterized in that** components (a) and (b) are in each case employed in amounts from 0.1 to 50% by weight - based on the weight of the gelatin capsules.

7. Use according to Claims 1 to 4, **characterized in that** components (a) and (b) are added to foodstuffs.

8. Use according to Claim 1, **characterized in that** components (a) and (b) are dispersed in butter, margarine, dietetic food, deep-frying oils, food oils, mayonnaises, salad dressings, cocoa products, sausage and the like.

## Revendications

1. Utilisation de mélanges de substances actives pour la fabrication d'agents hypocholestérolémiants,
**caractérisé en ce qu'**
on utilise
(a) des esters de β-silosténol ou de β-silostanol avec des acides carboxyliques répondant à la formule (I)
**R**^{**1**}**CO-OH** **(I)**
dans laquelle R¹CO représente un reste acyle aliphatique, linéaire ou ramifié, ayant de 2 à 22 atomes de carbone et O et/ou 1, 2 ou 3 doubles liaisons, et
(b) des acides gras ayant de 6 à 24 atomes de carbone et au moins deux doubles liaisons conjuguées ou leurs glycérides.

2. Utilisation selon la revendication 1,
**caractérisée en ce que**
comme composant (a) on utilise des esters de β-silosténol ou de β-silostanol avec des acides gras ayant de 12 à 18 atomes de carbone.

3. Utilisation selon les revendications 1 et/ou 2,
**caractérisée en ce que**
comme composant (b) on utilise de l'acide linoléique conjugué (CLA).

4. Utilisation selon les revendications 1 à 3,
**caractérisée en ce qu'**
on utilise les composants (a) et (b) dans le rapport de poids de 99:1 à 1:99.

5. Utilisation selon les revendications 1 à 4,
**caractérisée en ce qu'**
on encapsule les composants (a) et (b) dans de la gélatine.

6. Utilisation selon la revendication 5,
**caractérisée en ce qu'**
on utilise les composants (a) et (b) respectivement en quantités de 0,1 à 50 % en poids - rapportées au poids des capsules de gélatine.

7. Utilisation selon les revendications 1 à 4,
**caractérisée en ce qu'**
on ajoute
les composants (a) et (b) à des produits alimentaires.

8. Utilisation selon la revendication 1,
**caractérisée en ce qu'**
on disperse les composants (a) et (b) on dispose dans du beurre, de la margarine, des aliments diététiques, des huiles pour friture, des huiles de table, des mayonnaises, des sauces à salade, des produits à base de cacao, des saucisses et similaires.
